(19) Europäisches Patentamt
European Patent Office
Office européen des brevets

(11) Veröffentlichungsnummer: **0 000 741**
**B1**

(12) **EUROPÄISCHE PATENTSCHRIFT**

(45) Veröffentlichungstag der Patentschrift: **22.07.81**

(21) Anmeldenummer: **78100523.6**

(22) Anmeldetag: **27.07.78**

(51) Int. Cl.³: **C 07 D 209/28,**
A 61 K 31/405

(54) **Indolacetoxyacetylaminosäurederivate, Verfahren zu ihrer Herstellung und ihre Verwendung in Arzneimittel.**

(30) Priorität: **06.08.77 DE 2735537**

(43) Veröffentlichungstag der Anmeldung:
**21.02.79 Patentblatt 79/4**

(45) Bekanntmachung des Hinweises auf die Patenterteilung:
**22.07.81 Patentblatt 81/29**

(84) Benannte Vertragsstaaten:
**BE CH DE FR GB NL**

(56) Entgegenhaltungen:
**DE - A - 2 413 125**
**FR - A - 2 192 828**

(73) Patentinhaber: **Troponwerke GmbH & Co. KG**
**Berliner Strasse 156**
**D-5000 Köln 80 (DE)**

(72) Erfinder: **Boltze, Karl-Heinz, Dr.**
**Am Burgtor 23**
**D-5060 Berg.Gladbach 3 (DE)**
Erfinder: **Dell, Hans-Dieter, Dr.**
**Kalmuentener Strasse 5**
**D-5060 Berg. Gladbach 2 (DE)**
Erfinder: **Jacobi, Haireddin, Dr.**
**Finkenweg 2**
**D-5653 Leichlingen (DE)**
Erfinder: **Opitz, Wolfgang, Dr.**
**Silesiusstrasse 80**
**D-5000 Köln 80 (DE)**
Erfinder: **Schöllnhammer, Günter**
**Hackberg 21**
**D-5060 Berg. Gladbach 1 (DE)**
Erfinder: **Vollbrecht, Dieter, Dr.**
**Katterbachstrasse 105**
**D-5060 Berg. Gladbach 2 (DE)**

(74) Vertreter: **Dill, Erwin, Dr. et al,**
**c/o BAYER AG Zentralbereich Patente Marken und Lizenzen Bayerwerk**
**D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 741 B1

Indolacetoxyacetylaminosäurederivate, Verfahren zu ihrer Herstellung und ihre Verwendung in Arzneimitteln

Die vorliegende Erfindung betrifft neue Indolacetoxyacetylaminosäurederivate, Verfahren zu ihrer Herstellung sowie ihre Verwendung in Arzneimitteln, insbesondere in Entzündungshemmern und Antirheumatika.

Die Erfindung betrifft neue Indolacetoxyacetylaminoessigsäurederivate der allgemeinen Formel (I)

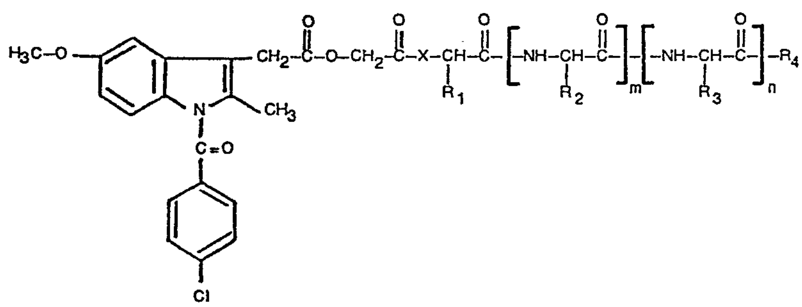

(I)

in welcher

m und n für die Ziffern 0 oder 1 stehen,

X für eine —HN— oder —$OCH_2$-Gruppe steht,

$R_1$, $R_2$ und $R_3$ gleich oder verschieden sind und jeweils für Wasserstoff, eine gegebenenfalls durch Hydroxy, Alkoxy mit 1 bis 4 Kohlenstoffatomen, Alkylthio mit 1 bis 4 Kohlenstoffatomen oder durch Phenalkyloxycarbonylamino mit 1 bis 4 Kohlenstoffatomen im Alkylteil, substituiertes Alkyl mit 1 bis 5 Kohlenstoffatomen, oder für eine Phenyl- oder Phenalkylgruppe mit 1 bis 4 Kohlenstoffatomen im Alkylteil, wobei die Phenylringe gegebenenfalls durch Trifluormethyl, Alkoxy mit 1 bis 4 Kohlenstoffatomen, Hydroxy oder Halogen substituiert sind oder für eine Heterocyclusmethylgruppe mit 5 bis 6 Ringgliedern, in welcher 1 oder 2 Glieder durch Stickstoff ersetzt sein können und an welche gegebenenfalls ein Benzolring annelliert ist, stehen oder für den Fall, daß m und n jeweils 0 bedeuten $R_1$ zusätzlich für eine Aminogruppe, eine Alkoxycarbonylaminogruppe oder eine Phenalkoxycarbonylaminogruppe mit jeweils 1 bis 5 Kohlenstoffatomen im Alkoxyteil steht,

$R_4$ für Hydroxy, Alkoxy mit 1 bis 4 Kohlenstoffatomen, Phenalkoxy mit 1 bis 4 Kohlenstoffatomen im Alkoxyteil, Amino, Alkylamino mit 1 bis 4 Kohlenstoffatomen im Alkylteil, Benzylamino oder Phenylamino steht,

sowie ihre gegebenenfalls mit Säuren und Basen gebildeten physiologisch verträglichen Salze.

Es wurde gefunden, daß man die erfindungsgemäßen Verbindungen der allgemeinen Formel (I), in welcher X, m, n und $R_1$ bis $R_4$ die oben angeführte Bedeutung haben, erhält, wenn man

a) [1-(4-Chlorbenzoyl)-5-methoxy-2-methyl-3-indolyl]acetoxyessigsäure (nachfolgend als ACE—OH bezeichnet) mit Chlorameisensäureestern der allgemeinen Formel

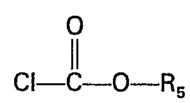

(II)

in welcher

$R_5$ für eine Alkylgruppe mit 1 bis 5 Kohlenstoffatomen steht,

zu gemischten Anhydriden der allgemeinen Formel (III)

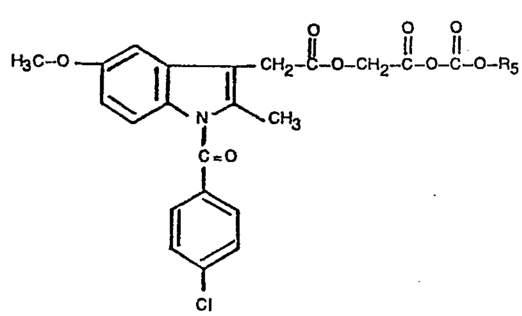

(III)

in welcher

$R_5$ die oben angegebene Bedeutung hat,

umsetzt und diese mit Verbindungen der allgemeinen Formel (IV)

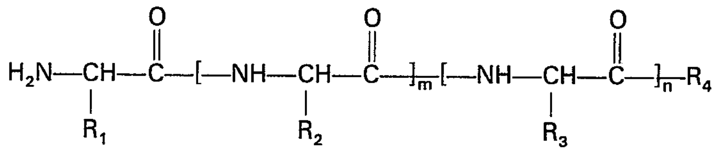

(IV)

in welcher

m, n und $R_1$ bis $R_4$ die in Anspruch 1 angeführte Bedeutung haben, zur Reaktion bringt, oder

b) die Verbindung ACE—OH mit einem entsprechend substituierten Phenol zu einem aktivierten Ester der allgemeinen Formel

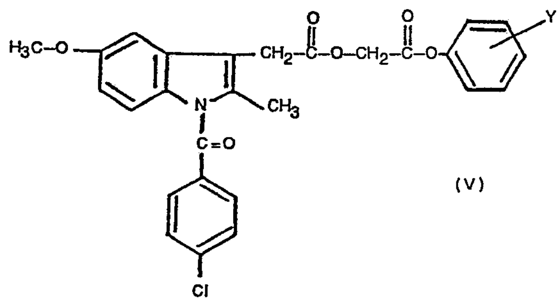

(V)

in welcher

Y für 2 bis 5 Halogenatome oder 1 bis 2 Nitrogruppen steht,

kondensiert und mit Verbindungen der allgemeinen Formel (IV) umsetzt oder

c) für den Fall, daß Hydroxylgruppen enthaltende Aminosäurederivate esterartig verknüpft werden sollen, die Verbindung ACE—OH mit Verbindungen der allgemeinen Formel (VI)

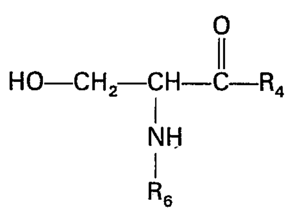

(VI)

in welcher

$R_4$ die oben angeführte Bedeutung hat und

$R_6$ für eine in der Peptidchemie übliche Aminoschutzgruppe steht,

umsetzt und anschließend den Rest $R_6$ in bekannter Weise abspaltet und gegebenenfalls die so erhaltenen Verbindungen der allgemeinen Formel (I) mit Säuren und Basen in die physiologisch verträglichen Salze verwandelt.

Für den Fall, daß die Verbindungen der allgemeinen Formel (I) eine freie Säuregruppe enthalten ($R_4 = OH$), können mit organischen und anorganischen Basen physiologisch verträgliche Additionssalze gebildet werden.

Als Beispiele solcher Salze seien vorzugsweise genannt Natriumsalze-, Kalium-, Magnesium-, Ammonium-, Methylammonium-, Dimethylammoniumsalze oder auch das 2-Hydroxyethylammoniumsalz.

Für den Fall, daß die erfindungsgemäßen Verbindungen der allgemeinen Formel (I) eine freie Aminogruppe aufweisen, können ebenfalls mit Säuren physiologisch verträgliche Additionssalze gebildet werden.

Als Beispiele solcher Salze seien genannt Halogenide, vorzugsweise Chloride, Sulfate, Citrate, Tartrate, Maleinate und Sulfonate.

Überraschenderweise zeigen die erfindungsgemäßen Verbindungen der allgemeinen Formel (I) eine vorteilhaftere Wirksamkeit als die aus dem Stand der Technik bekannten Indometacinserinderivate der DOS 2 413 125, wie aus der folgenden Tabelle hervorgeht.

In dieser Tabelle werden die erfindungsgemäßen Aminosäurederivate den entsprechenden Aminosäurederivaten des Indometacins gegenübergestellt. Als pharmakologisches Testmodell zur Bestimmung der entzündungshemmenden Wirkung wurde das Kaolinödem an der Rattenpfote (vgl. KEMPER et al, Z. ges. exp. Med. *131* (1959), 407) verwendet. Hierbei zeigte es sich, daß die erfindungsgemäßen Aminosäurederivate überraschenderweise eine deutlich stärkere Wirkung zeigen

als die aus dem Stand der Technik bekannten Aminosäurederivate des Indometacins (vgl. DT—OS 2 413 125).

Die Applikation der Wirkstoffe erfolgte an Ratten oral eine halbe Stunde vor der Kaolinapplikation (Dosis: s. Spalten 2 und 4). Die Hemmung wurde 4 Stunden nach der Ödemprovokation gegenüber Kontrollgruppen bestimmt (s. Spalten 3 und 5).

TABELLE

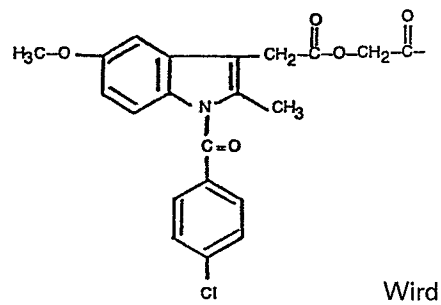

Wird im folgenden als ACE bezeichnet

| Derivat | Indometacin | | ACE—OH | | |
|---|---|---|---|---|---|
| 1 | 2 | 3 | 4 | 5 | Beispiel-Nr. |
| | Dosis mg/kg | % Hemmung | Dosis mg/kg | % Hemmung | |
| Serin | 100 | 34,3 | 12,5 | 56 | 2 |
| Serinbenzylester | 50 | 17,2 | 50 | 41,9 | 14 |
| Tyrosin | 50 | 25,4 | 12,5 | 48 | 4 |
| Tryptophan | 100 | 35,5 | | | 5 |
| Tryptophan | 50 | 13 | 6,25 | 24 | 5 |
| Alanin | 100 | 0 | 50 | 46 | 8 |
| Phenylglycin | 100 | 35 | | | 9 |
| Phenylglycin | 50 | 0,7 | 25 | 42 | 9 |

Weiterhin wurde das erfindungsgemäße Serinderivat mit dem bekannten Serinderivat des Indometacins verglichen, indem jeweils die $DE_{50}$ ermittelt wurde. Hierbei zeigt sich, daß das erfindungsgemäße Serinderivat bei Ratten nach oraler Applikation eine $DE_{50}$ von 2,14 mg/kg Körpergewicht und bei subkutaner Applikation eine $DE_{50}$ von 2,17 mg/kg Körpergewicht besitzen. Die entsprechenden $DE_{50}$ — Werte des Serinderivates von Indometacin lagen in jedem Fall über 100 mg/kg Körpergewicht.

Es wurden eine weitere Anzahl von Aminosäurederivaten des Indometacins, die bisher noch nicht bekannt waren, hergestellt und in dem obengenannten Testmodell zur Bestimmung der Entzündungshemmung geprüft. Hierbei zeigte sich, daß diese Derivate nach oraler Verabreichung von 50 bzw. 100 mg-Dosen nur eine sehr geringe oder keine entzünungshemmende Wirkung besitzen, wie aus der folgenden Aufstellung ersichtlich ist.

Glutaminsäurederivat (178—180°C; 100 mg-kg; 3,5%);
Glutaminsäuredibenzylesterderivat (143—144°C; 50 mg/kg; 6,6%);
Tyrosinbenzylesterderivat (124—126°C; 100 mg/kg; 0,0%);
Phenylglycinbenzylesterderivat (148—151°C; 100 mg/kg; 0,0%);
Tryptophenbenzylesterderivat (164—165°C; 100 mg/kg; 0,0%);
Histidinderivat (230°C/Zers.; 50 mg/kg; 10,6%);
N-Methylglycinderivat (165—166°C; 100 mg/kg; 21%);
Histindinbenzylesterderivat (218—219°C; 100 mg/kg; 0,0%);
(in Klammern die Angaben für Schmelzpunkt, Menge der oral verabreichten Substanz und Ödemhemmung in %).

Demgegenüber zeigen die erfindungsgemäßen Aminosäurederivate überraschenderweise eine deutlich bessere entzündungshemmende Wirkung, wie aus der folgenden Aufstellung ersichtlich ist.

N—ACE-Phenylalanyltyrosinmethylester (Beispiel 19; 12,5 mg/kg; 52,8%);
N—ACE-Glycylalanylphenylalanylmethylester (Beispiel 20; 50 mg/kg; 42%);
N—ACE-Phenylalanin $DE_{50}$ (Beispiel 7; 5,1 mg/kg);
N—ACE-Methionin (Beipiel 3; 25 mg/kg; 49,7%);
N—ACE-O-Methylserin $DE_{50}$ (Beispiel 10; 27,9 mg/kg);
N—ACE-Serinmethylester $DE_{50}$ (Beispiel 1; 19,7 mg/kg);
O—ACE-Serinethylester (Beispiel 18; 12,5 mg/kg; 49,7%);
O—ACE-Serinmethylester (Beispiel 16; 25 mg/kg; 51,1%).

Diese Testergebnisse zeigen eindeutig die überraschend vorteilhafte Wirkung der erfindungsgemäßen Aminosäurederivate. Bei Kenntnis des Standes der Technik konnte nicht erwartet werden, daß die Einführung eines Aminosäureesters in die ACE-Verbindung einen so sprunghaften Wirkungsanstieg verursacht. Man hätte vielmehr erwarten müssen, daß die Wirkung der erfindungsgemäßen Verbindungen mit der Wirkung der entsprechenden Indometacin-Aminosäurederivate vergleichbar wäre.

Verwendet man ACE—OH und Chlorameisensäureester als Ausgangsverbindungen, so kann der Reaktionsablauf der Variante a) durch das folgende Formelschema wiedergegeben werden:

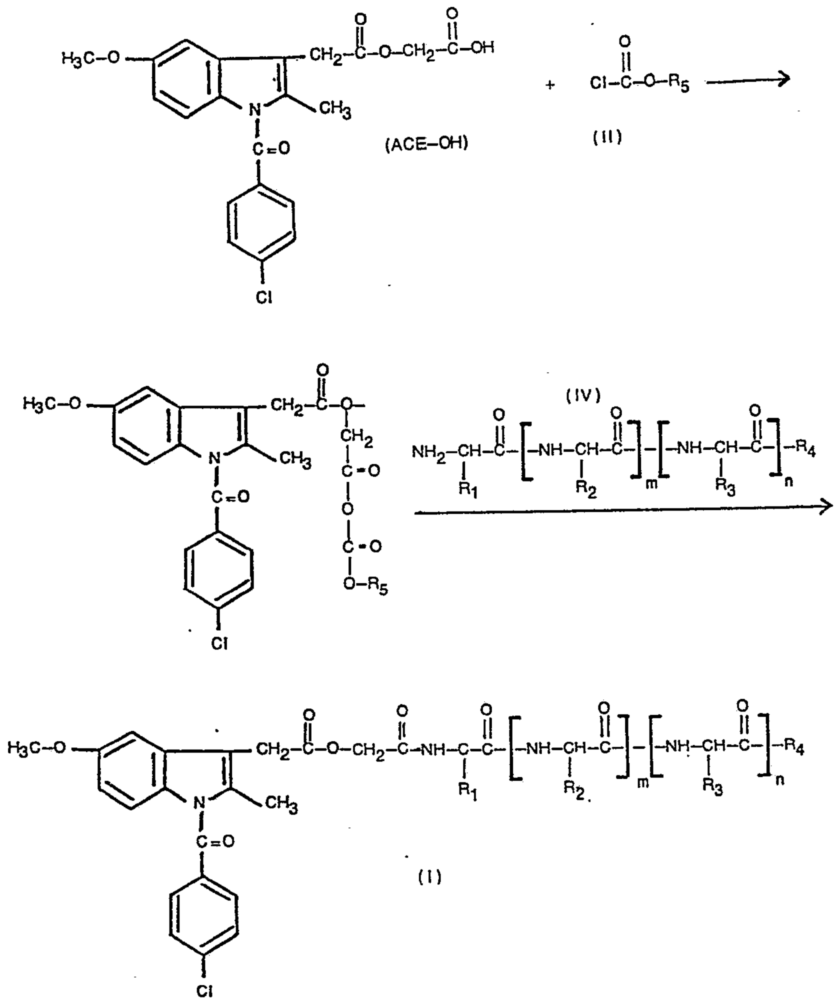
(ACE—OH)
(II)
(IV)
(I)

Die als Ausgangsverbindungen verwendbaren Chlorameisensäureester der allgemeinen Formel (II) sind bekannt und die als Ausgangsverbindung verwendete ACE—OH ist ebenfalls bekannt (DOS 2 234 651).

Die als Zwischenprodukt gebildeten gemischten Anhydride der allgemeinen Formel (III) sind neu, können aber nicht indentifiziert werden, da sie sich — wie auch der überwiegende Teil der literaturbekannten gemischten Anhydride — bei der Aufarbeitung zersetzen (vgl. Houben-Weyl, Methoden der Organischen Chemie, 4. Auflage, Band 15/2, Seite 17, 3. Absatz).

Die als Ausgangsverbindungen verwendbaren Verbindungen der allgemeinen Formel (IV) sind weitgehend literaturbekannt oder können nach bekannten Methoden hergestellt werden (vgl. u.a. Houben-Weyl, Methoden der organischen Chemie, 4. Auflage, Band 15/1 und 2).

Die Reaktion erfolgt zweckmäßig in Gegenwart von Verdünnungsmitteln. Als Verdünnungsmittel kommen inerte organische Lösungsmittel in Frage, insbesondere solche, die polar aprotisch sind. Beispielhaft seien genannt: Tetrahydrofuran, Dioxan, Dimethylformamid, Hexamethylphosphorsäuretriamid.

Die Reaktion erfolgt zweckmäßig in Gegenwart von Säurebindern. Als Säurebinder können alle üblichen Mittel verwendet werden, welche den freiwerdenden Chlorwasserstoff zu binden vermögen. Hierzu gehören vorzugsweise Alkalihydroxide und -carbonate oder organische Basen wie Pyridin und Triethylamin.

Die Reaktionstemperaturen liegen zweckmäßigerweise unterhalb von 0°C, da andernfalls Racemisierungen auftreten; sie können in einem gewissen Bereich variiert werden.

Im allgemeinen arbeitet man bei Temperaturen zwischen 0 bis −25°C, vorzugsweise zwischen −10 und −20°C.

Die Umsetzung erfolgt üblicherweise bei Normaldruck.

Bei der Durchführung des erfindungsgemäßen Verfahrens setzt man vorzugsweise auf 1 Mol ACE—OH, 1 Mol Chlorameisensäureester, 1 Mol des Aminosäurederivates sowie 1 Mol des Säurebinders ein.

Die Reaktionszeit liegt zwischen 6 und 24 Stunden, vorzugsweise bei 14 bis 20 Stunden.

Die Aufarbeitung erfolgt vorzugsweise durch Eindampfen im Vakuum, Aufnehmen in eins der üblichen organischen Lösungsmittel, Neutralwaschen, gegebenenfalls Reinigen an Kieselgel und Rekristallisation.

Verwendet man für die Reaktion anstelle eines gemischten Anhydrids von ACE—OH einen aktivierten Ester der allgemeinen Formel (V), so kann die Reaktion der Variante b) durch das folgende Reaktionsschema wiedergegeben werden:

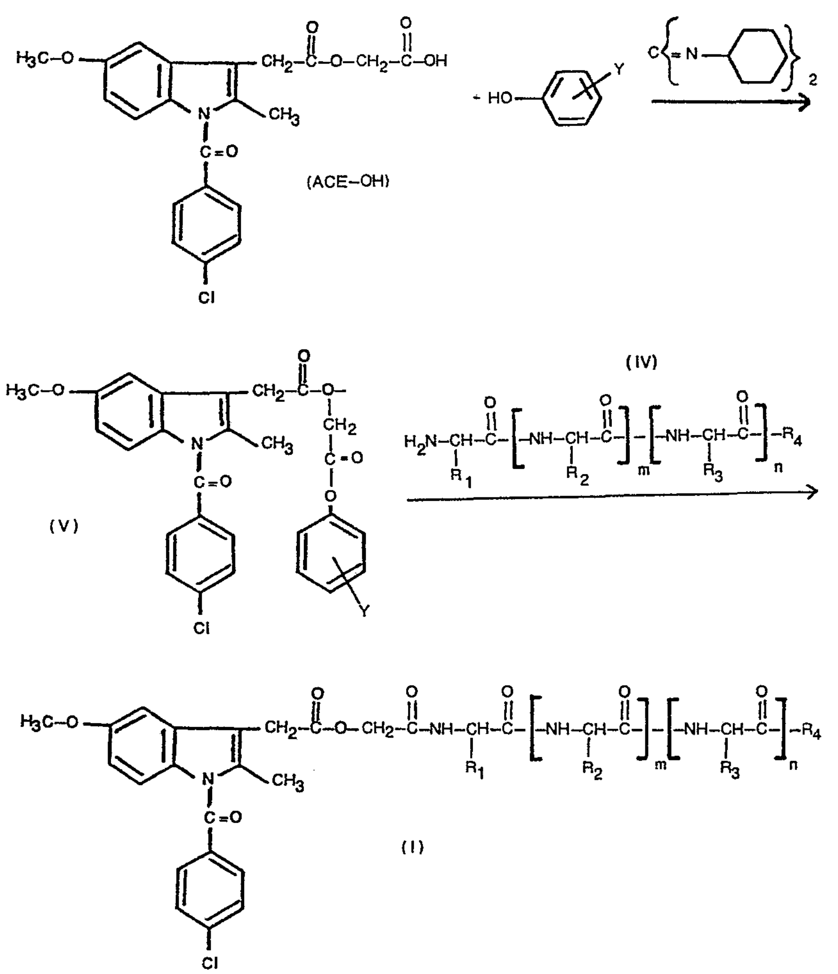

(ACE–OH)

(IV)

(V)

(I)

Die als Reaktionspartner verwendeten Verbindungen der allgemeinen Formel (V) sind bisher nicht bekannt, können aber in an sich bekannter Weise durch Umsetzung von 1 Mol ACE—OH und 1 Mol des entsprechend substituierten Phenols in einem polar aprotischen organischen Lösungsmittel, wie z.B. Tetrahydrofuran, in Gegenwart von 1 Mol eines Carbodiimids, wie z.B. Dicyclohexylcarbodiimid oder Carbonyldiimidazol in etwa 1 1/2-stündiger Reaktionszeit bei Raumtemperatur erhalten werden.

Das erfindungsgemäße Verfahren (V) → (I) erfolgt zweckmäßig in Gegenwart von Verdünnungsmitteln. Als Verdünnungsmittel kommen die für diese Reaktion literatur-bekannten organischen Lösungsmittel, vorzugsweise Dimethylformamid, Dioxan, Pyridin oder deren Gemische mit Wasser in Frage.

Soweit Additionssalze von (IV) zu Reaktionen verwendet werden, erfolgt das Verfahren zweckmäßig in Gegenwart von Säurebindern. Beispielhaft seien genannt: Alkalihydroxide, -carbonate oder starke organische Amine, vorzugsweise Triethylamin.

Die Reaktionstemperaturen können in einem gewissen Bereich variiert werden. Normalerweise arbeitet man bei 15 bis 25°C, da bei niedrigeren Temperaturen die Reaktionszeiten unverhältnismäßig verlängert werden, bei höheren Temperaturen jedoch die Racematbildung gefördert wird. Eine gewisse Racematbildung ist auch bei der Reaktion bei Raumtemperatur nicht auszuschließen./

Das Verfahren wird üblicherweise bei Normaldruck durchgeführt.

Bei der Durchführung des erfindungsgemäßen Verfahrens wird vorzugsweise pro Mol des aktivierten Esters (V) 1 Mol des Aminosäurederivates (IV) eingesetzt. Die Aufarbeitung erfolgt zweckmäßigerweise durch Verdünnen des Ansatzes mit Wasser, Aufnehmen in einem geeigneten organischen Lösungsmittel, Neutralisieren, Eindampfen und Rekristallisieren aus geeigneten Lösungsmitteln.

Für den Fall, daß die Aminosäurederivate der allgemeinen Formel (VI) esterartig mit ACE—OH verknüpft werden sollen, kann die Reaktion der Variante c) durch das nachfolgende reaktionsschema wiedergegeben werden:

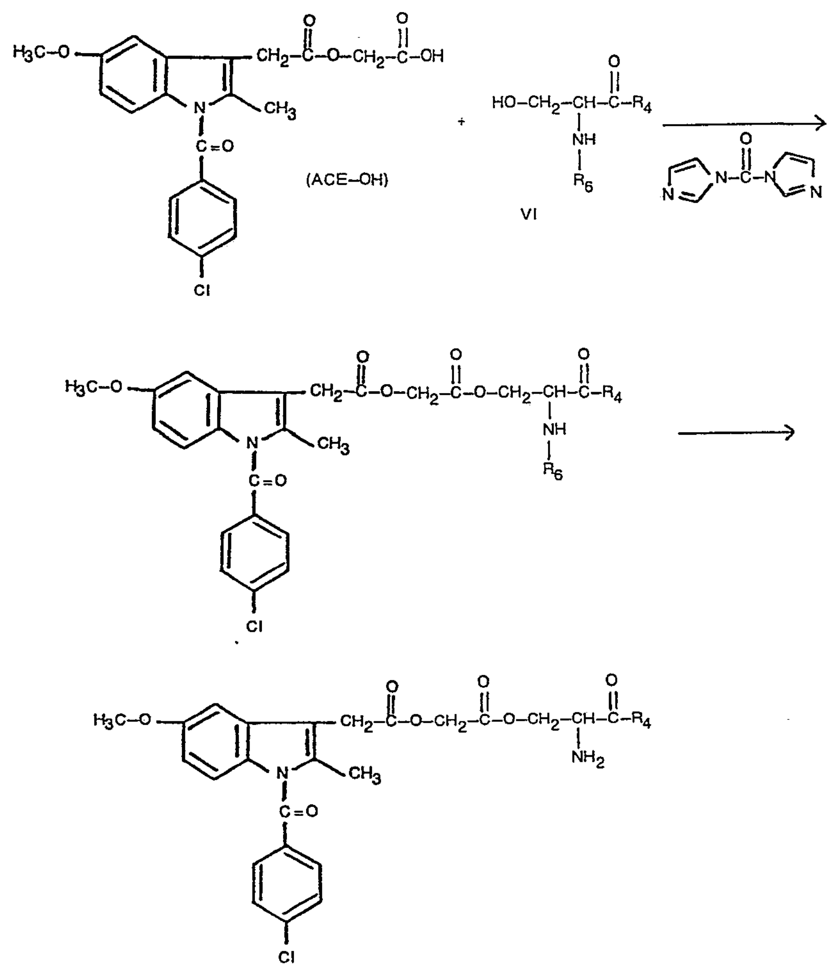


Die als Ausgangsverbindungen verwendeten Serinderivate der allgemeinen Formel (VI) sind bekannt. Die Reaktion erfolgt vorzugsweise in Gegenwart von Verdünnungsmitteln. Als Verdünnungsmittel können polar aprotische organische Lösungsmittel verwendet werden. Beispielhaft seien genannt Tetrahydrofuran, Dioxan, Dimethylformamid, Hexamethylphosphorsäuretriamid.

Die erfindungsgemäße Reaktion erfolgt vorzugsweise in Gegenwart eines wasserentziehenden Mittels. Beispielhaft seien genannt Dicyclohexylcarbodiimid oder Carbonyldiimidazol in Gegenwart katalytischer Mengen eines basischen Katalysators, vorzugsweise Natriumhydrid.

Die Reaktionstemperaturen können in größerer Breite variiert werden; üblicherweise arbeitet man zwischen 10 und 40°C, vorzugsweise zwischen 15 und 25°C.

Zweckmäßigerweise bringt man zunächst 1 Mol der ACE—OH mit 1 Mol Carbonyldiimidazol zur Reaktion und fügt 1 Mol des Aminosäurederivates erst nach beendeter $CO_2$-Entwicklung hinzu. Die Aufarbeitung erfolgt in üblicher Weise, vorzugsweise durch Eindampfen im Vakuum, Aufnehmen in ein geeignetes Lösungsmittel und Reinigen an Kieselgel. Die Abspaltung der Aminoschutzgruppe $R_6$ erfolgt nach den in der Peptidchemie üblichen literaturbekannten Methoden.

Als neue Wirkstoffe seien beispielhaft genannt:

N—ACE-Serinmethylester;
N—ACE-Serin;
N—ACE-Methionin;
N—ACE-Tyrosin;
N—ACE-Tryptophan;
N—ACE-DL-Threonin;
N—ACE-Phenylalanin;
N—ACE-Serin-N'-methylamid;
N—ACE-Histidinmethylester;
N—ACE-Serinbenzylester;
O—ACE-Serinmethylester;
O—ACE-Serinethylester;
N—ACE-Phenylalanyltyrosinmethylester;
N—ACE-Glycylalanylphenylalaninmethylester;
N-$\alpha$-ACE—N-$\gamma$-benzyloxycarbonyldiaminobutyrylphenylalanylphenylalaninmethylester.

Als ACE sei hier und im weiteren Verlauf der Rest

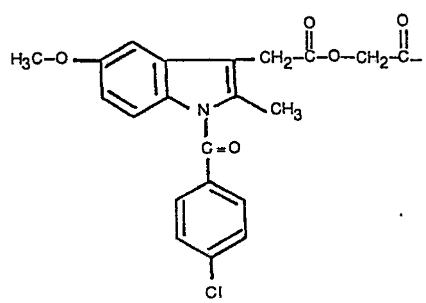

verstanden.

Die neuen Wirkstoffe können in bekannter Weise in die üblichen Formulierungen überführt werden wie Tabletten, Kapseln, Dragees, Pillen, Granulate, Aerosole, Sirupe, Emulsionen, Suspensionen, Lösungen, Salben, Gele, Cremes, Gallerten, unter Verwendung inerter, nichttoxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Falle der Benutzung von Wasser als Verdünnungsmittel gegebenenfalls organische Lösungsmittel als Hilfslösungsmittel verwendet werden können.

Als Hilfsstoffe seien beispielhaft aufgeführt:

Wasser, nichttoxische organische Lösungsmittel wie Paraffine (z.B. Erdölfraktionen), pflanzliche Ole (z.B. Erdnuß-/Sesamöl), Alkohole (z.B. Ethylalkohol, Glycerin), Glykole (z.B. Propylenglykol, Polyethylenglykol); feste Trägerstoffe wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Roh-, Milch- und Traubenzucker); Emulgiermittel wie nichtionogene und anionische Emulgatoren (z.B. Polyoxyethylen-Fettsäureester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natrimlaurylsulfat).

Die Applikation erfolt in üblicher Weise, vorzugsweise oral oder parenteral, lokal, intramuskulär oder intravenös.

Im Falle der oralen Anwendung können Tabletten, selbstverständlich außer den genannten Trägerstoffen auch Zusätze wie Natriumcitrat, Calciumcarbonat und Dicalciumphosphat, zusammen mit verschiedenen Zuschlagstoffen wie Stärke, vorzugsweise Kartoffelstärke, Gelatine und dergleichen enthalten.

Weiterhin können Gleitmittel wie Magnesiumstearat, Natriumlaurylsulfat und Talkum zum Tablettieren mitverwendet werden. Im Falle wäßriger Suspensionen und/oder Elixiere, die für orale Anwendungen gedacht sind, können die Wirkstoffe außer mit den genannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Für den Fall der lokalen Anwendung kommen vorzugsweise Lösungen, Emulsionen, Salben, Gele, Cremes, Aerosole oder Gallerten mit geeigneten vorgenannten Hilfsstoffen in Betracht.

Im allgemeinen hat es sich als vorteilhaft erwiesen, bei oraler Applikation Mengen von etwa 1 bis 100 mg, insbesondere 10 bis 40 mg pro Dosis bei 2- bis 3-maliger täglicher Verabreichung zur Erzielung wirksamer Ergebnisse einzusetzen, bei rektaler Applikation beträgt die Dosierung etwa 10 bis 200, insbesondere 30 bis 80 mg pro Dosis.

Beispiel 1

*N—ACE-Serinmethylester*

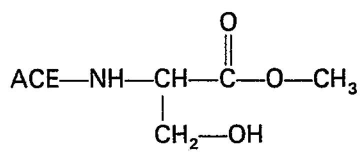

5 g (0,012 Mol) ACE—OH werden in 50 ml absolutem Tetrahydrofuran gelöst, mit 1,8 ml (0,0131 Mol) Triethylamin versetzt, auf −10°C gekühlt und während einer halben Stunde tropfenweise mit 1,7 ml (0,0131 Mol) Chlorameisensäuren-n-butylester versetzt. Das so gebildete gemischte Anhydrid ACE—O—COO-n-$C_4H_9$ kann nicht isoliert werden, da es sich bereits bei Raumtemperatur zersetzt. Es wird — wie auch in den weiteren Beispielen — in Lösung weiterverarbeitet, indem man 2,05 g (0,0131 Mol) L-Serinmethylester, gelöst in 20 ml Dimethylformamid und weiter 1,8 ml Triethylamin bei −10°C und weiteren 12-stündigem Stehen bei −18°C wird der Ansatz bei 3 Torr und 30°C eingedampft, der Rückstand in Chloroform gelöst, mit 2 n HCl und dann mit Wasser neutralgewaschen, über $Na_2SO_4$ getrocknet, filtriert und eingedampft. Nach Reinigen an Kieselgel mit einem Gemisch aus Chloroform/Methanol, Eindampfen, Aufnehmen des Rückstandes in 50 ml Ethanol und Zufügen von Wasser bis zur beginnenden Kristallisation werden 5,05 g ACE-Serinmethylester (=82,1% der Theorie) vom Fp. 88°C erhalten.

$[\alpha]_D^{20} = -2{,}6$ (c = 0,5; Dimethylformamid).

Für $C_{25}H_{25}ClN_2O_8$

| | | | | |
|---|---|---|---|---|
| bereichnet: | C 58,09%, | H, 4.87%, | Cl 6,86%, | N 5,42%; |
| gefunden: | C 58,05%, | H 4,94%, | Cl 6,75%, | N 5,43%. |

In gleicher Weise können hergestellt werden:

Beispiel 2

*N—ACE-Serin*

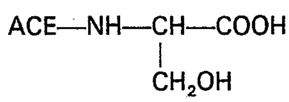

mit L-Serin; Fp. 133°C, Ausbeute 70,8% der Theorie.

$[\alpha]_D^{20} = +6{,}8$ (c = 0,5; Dimethylformamid).

Für $C_{24}H_{23}ClN_2O_8$

| | | | | |
|---|---|---|---|---|
| berechnet: | C 57,32%, | H 4,61%, | Cl 7.05%, | N 5,57%; |
| gefunden: | C 57,22%, | H 4,59%, | Cl 7,22%, | N 5,51%. |

Beispiel 3

*N—ACE-Methionin*

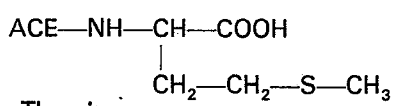

Fp. 165°C, Ausbeute 70,1% der Theorie.

$[\alpha]_D^{20} = -0{,}8$ (c = 0,5; Dimethylformamid).

Für $C_{26}H_{27}ClN_2O_7S$

| | | | | |
|---|---|---|---|---|
| berechnet: | C 57,09%, S 5,86%; | H 4,97%, | Cl 6,48%, | N 5,12%, |
| gefunden: | C 57,26%, S 5,97%. | H 5,01%, | Cl 6,74%, | N 5,24%, |

Beispiel 4

*N—ACE-Tyrosin*

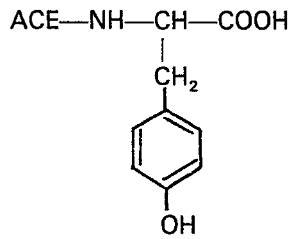

Fp. 111 °C, Ausbeute 34,7% der Theorie.
$[\alpha]_D^{20} = +7,3$ (c = 0,5; Dimethylformamid).
Für $C_{30}H_{27}ClN_2O_8$

| | | | | |
|---|---|---|---|---|
| berechnet: | C 62,23%, | H 4,70%, | Cl 6,12%, | N 4,84%; |
| gefunden: | C 62,33%, | H 4,70%, | Cl 6,00%, | N 4,78%. |

Beispiel 5

*N—ACE-Tryptophan*

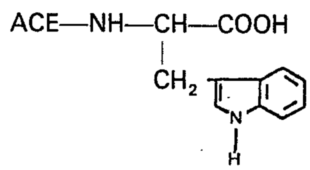

sintert bei 120 bis 150 °C, Ausbeute 65,3% der Theorie.
$[\alpha]_D^{20} = +9,4$ (c = 0,5; Dimethylformamid).
Für $C_{32}H_{28}ClN_3O_7$

| | | | | |
|---|---|---|---|---|
| berechnet: | C 63,84%, | H 4,69%, | Cl 5,89%, | N 6,98%; |
| gefunden: | C 64,76%, | H 4,72%, | Cl 5,82%, | N 6,94%. |

Beispiel 6

*N—ACE—DL-Threonin*

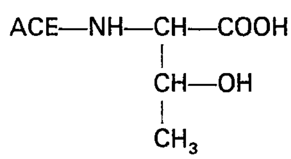

Fp. 119 °C, Ausbeute 47% der Theorie.
$[\alpha]_D^{20} = -0,8$ (c = 0,5; Dimethylformamid).
Für $C_{25}H_{25}ClN_2O_8$

| | | | | |
|---|---|---|---|---|
| berechnet: | C 58,09%, | H 4,87%, | Cl 6,86%, | N 5,42%; |
| gefunden: | C 57,96%, | H 4,84%, | Cl 6,77%, | N 5,56%. |

Beispiel 7

*N—ACE-Phenylalanin*

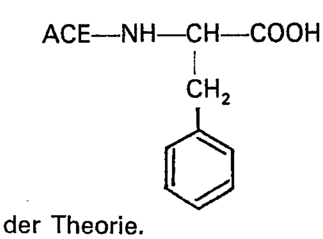

Fp. 174 °C, Ausbeute 57,8% der Theorie.
$[\alpha]_D^{20} = +4,9$ (c = 0,5; Dimethylformamid).
Für $C_{30}H_{27}ClN_2O_7$

| | | | | |
|---|---|---|---|---|
| berechnet: | C 64,00%, | H 4,83%, | Cl 6,30%, | N 4,98%; |
| gefunden: | C 64,14%, | H 4,83%, | Cl 6,28%, | N 5,06%. |

Beispiel 8

*N—ACE-Alanin*

ACE—NH—CH—COOH
|
$CH_3$

Fp. 140°C, Ausbeute 48% der Theorie.
$[\alpha]_D^{20} = -4,6$ (c = 0,5; Dimethylformamid).
Für $C_{24}H_{23}ClN_2O_7$

| | | | | |
|---|---|---|---|---|
| berechnet: | C 59,20%, | H 4,76%, | Cl 7,28%, | N 5,76%; |
| gefunden: | C 59,32%, | H 4,83%, | Cl 7,15%, | N 5,73%. |

Beispiel 9

*N—ACE-Phenylglycin*

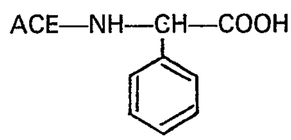

Fp. 179 bis 182°C, Ausbeute 57,6% der Theorie.
$[\alpha]_D^{20} = +61,8$ (c = 0,5; Dimethylformamid).
Für $C_{29}H_{25}ClN_2O_7$

| | | | | |
|---|---|---|---|---|
| berechnet: | C 63,45%, | H 4,59%, | Cl 6,46%, | N 5,10%; |
| gefunden: | C 63,61%, | H 4,57%, | Cl 6,44%, | N 5,03%. |

Beispiel 10

*N—ACE—DL—O-Methylserin*

ACE—NH—CH—COOH
|
$CH_2$—O—$CH_3$

Fp. 96°C, Ausbeute 39% der Theorie.
$[\alpha]_D^{20} = 0$ (c = 0,5%; Dimethylformamid).
Für $C_{25}H_{25}ClN_2O_8$

| | | | | |
|---|---|---|---|---|
| berechnet: | C 58,09%, | H 4,87%, | Cl 6,86%, | N 5,42%; |
| gefunden: | C 57,89%, | H 4,91%, | Cl 6,74%, | N 5,46%. |

Beispiel 11

*N—ACE—DL-Serinmethylamid*

ACE—NH—CH—CONH—$CH_3$
|
$CH_2$—OH

Fp. 141°C, Ausbeute 42,3% der Theorie.
$[\alpha]_D^{20} = +3,2$ (c = 0,5; Dimethylformamid).
Für $C_{25}H_{26}ClN_3O_7$

| | | | | |
|---|---|---|---|---|
| berechnet: | C 58,20%, | H 5,08%, | Cl 6,87%, | N 8,14%; |
| gefunden: | C 58,19%, | H 5,16%, | Cl 6,85%, | N 8,13%. |

Beispiel 12

*ACE-2,4,5-Trichlorphenylester*

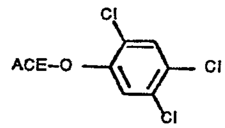

2 g (0,0048 Mol) ACE—OH und 1 g (0,0051 Mol) 2,4,5-Trichlorphenol werden in 25 ml Tetrahydrofuran gelöst, mit 1 g (0,0048 Mol) Dicyclohexylcarbodiimid versetzt und 1,5 Stunden bei Raumtemperatur gerührt. Anschließend wird der ausgefallene Dicyclohexylharnstoff abgesaugt, die Lösung eingedampft und mit 20 ml Ethanol verrieben. Nach beendeter Kristallisation wird abgesaugt und der Kristallbrei mit Ethanol und Petrolether gewaschen.

Fp. 132 bis 134°C, Ausbeute 2,3 g = 80,4% der Theorie.

Es handelt sich um ein Zwischenprodukt für die Herstellung der folgenden Verbindungen.

Beispiel 13

*N—ACE-Histidinmethylester*

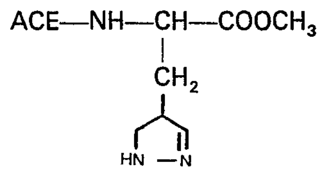

4,86 g (0,02 Mol) L-Histidinmethylesterdihydrochlorid und 8,3 ml (0,06 Mol) Triethylamin werden in 100 ml Dimethylformamid gelöst und bei −10°C mit 5,67 g (0,02 Mol) der im Beispiel 12 beschriebenen Verbindung versetzt.

Nach mehrstündigem Rühren und Stehen bei Raumtemperatur wird der Ansatz mit Wasser verdünnt und mehrfach mit Ethylenchlorid extrahiert. Die Ethylenchloridlösung wird mit nHCl ausgeschüttelt und mit Wasser neutralgewaschen. Die organische Phase wird anschließend über $Na_2SO_4$ getrocknet, filtriert und eingedampft. Der Rückstand wird in Chloroform/Methanol (9:1) an Kieselgel gereinigt.

Fp. 119°C, Ausbeute 3,7 g = 34% der Theorie.

$[\alpha]_D^{20} = +3,5$ (c = 3,5; Dimethylformamid).

Für $C_{28}H_{27}ClN_4O_7$

| | | | | |
|---|---|---|---|---|
| berechnet: | C 59,31%, | H 4,80%, | Cl 6,25%, | N 9,88%; |
| gefunden: | C 59,37%, | H 5,01%, | Cl 6,18%, | N 9,82%. |

Beispiel 14

*N—ACE-Serinbenzylester*

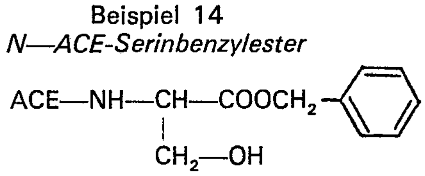

analog Beispiel 13 unter Verwendung von L-Serinbenzylester.

Fp. 158 bis 159°C, Ausbeute 67% der Theorie.

$[\alpha]_D^{20} = 10,9$ (c = 0,5; Dimethylformamid).

Für $C_{31}H_{29}ClN_2O_8$

| | | | | |
|---|---|---|---|---|
| berechnet: | C 62,78%, | H 4,93%, | Cl 5,98%, | N 4,72%; |
| gefunden: | C 62,93%, | H 5,01%, | Cl 6,15%, | N 4,72%. |

Beispiel 15

*O—ACE—N-Boc-Serinmethylester*

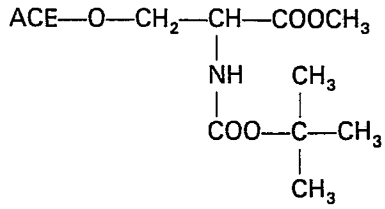

4,15 g (0,01 Mol) ACE—OH werden in 15 ml Tetrahydrofuran gelöst, mit 1,78 g (0,011 Mol) 1,1'-Carbodiimidazol versetzt und bis zur Beendigung der $CO_2$-Entwicklung gerührt.

In dieser Lösung wird eine Lösung von 2,25 g (0,01 Mol) N-Boc-Serinmethylester in 10 ml Tetrahydrofuran in Gegenwart katalytischer Mengen von Natriumhydrid hinzugefügt und 7 Stunden bei 35°C gerührt. Anschließend wird die Lösung bei 3 Torr eingedampft, der Rückstand wird in Ethylacetat aufgenommen und an Kieselgel gereinigt. Die Verbindung ist ein Öl. Ausbeute 5,1 g (=82% der Theorie).

Beispiel 16

*O—ACE-Serinmethylestertrifluoracetat*

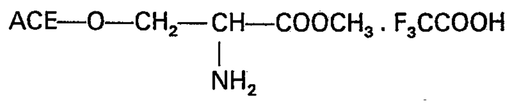

Die Verbindung entsteht in bekannter Weise durch Versetzen der Verbindung aus Beispiel 15 mit Trifluoressigsäure.

Fp. 69 bis 70°C, Ausbeute 65% der Theorie.

$[\alpha]_D^{20} = +0,8$ (c = 0,5; Dimethylformamid).

Für $C_{25}H_{25}ClN_2O_8 . C_2HF_3O_2$

| | | | | |
|---|---|---|---|---|
| berechnet: | C 51,40%, N 4,44%; | H 4,15%, | Cl 5,62%, | F 9,03%, |
| gefunden: | C 51,52%, N 4,54%. | H 4,22%, | Cl 5,52%, | F 8,8 %, |

Beispiel 17

*O—ACE—N-Boc-Serinethylester*

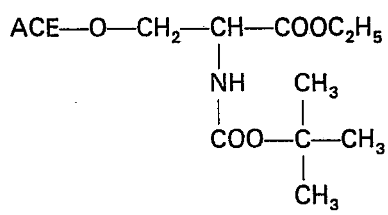

analog Beispiel 15 aus 10,15 g ACE—OH und 5,7 g Boc-Serinethylester.

Fp. 99 bis 100°C, Ausbeute 6,2 g (Rohprodukt).

Beispiel 18

*O—ACE-Serinethylestertrifluoracetat*

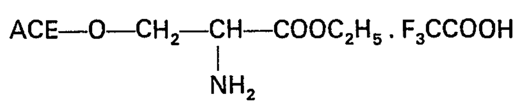

Analog Beispiel 16 aus der in Beispiel 17 beschriebenen Verbindung.

Fp. 56 bis 58°C, Ausbeute 77% der Theorie.

$[\alpha]_D^{20} = -2,4$ (c = 0,5; Dimethylformamid).

Für $C_{26}H_{27}ClN_2O_8 . C_2HF_3O_2$

| | | | | |
|---|---|---|---|---|
| berechnet: | C 52,14%, N 4,34%; | H 4,38%, | Cl 5,50%, | F 8,84%, |
| gefunden: | C 52,21%, N 4,37%. | H 4,36%, | Cl 6,30%, | F 8,68%, |

Beispiel 19

*N—ACE-Phenylalanyltyrosinmethylester*

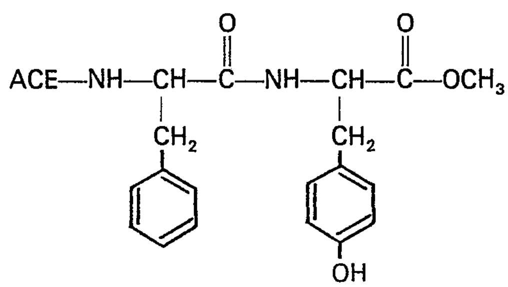

Eine Suspension von 0,005 Mol Phenylalanyltyrosinmethylestertrifluoracetat in 15 ml absolutem Dimethylformamid wird unter Rühren bei 0°C zunächst mit 0,005 Mol Triethylamin und nach 10 Minuten mit 0,005 Mol ACE-2,4,5-trichlorphenylester versetzt. Nach 2-stündigem Rühren, zuletzt bei Raumtemperatur, wird das Reaktionsgemisch mit 150 ml Wasser versetzt, dreimal mit Ethylacetat extrahiert und die organische Phase dreimal mit nHCl-Lösung ausgeschüttelt. Anschließend wird die Lösung mit Wasser neutralgewaschen, über $Na_2SO_4$ getrocknet und eingedampft. Nach Rekristallisation aus Aceton/Petrolether werden 84% der Theorie an N—ACE-Phenylalanyltyrosinmethylester vom Fp. 131 bis 132°C erhalten.

$[\alpha]_D^{20} = -5{,}2$ (c = 0,5; Dimethylformamid).

Für $C_{40}H_{38}ClN_3O_9$

| | | | | |
|---|---|---|---|---|
| berechnet: | C 64,90%, | H 5,17%, | Cl 4,79%, | N 5,68%; |
| gefunden: | C 64,87%, | H 5,36%, | Cl 5,20%, | N 5,63%. |

Beispiel 20

*ACE-Glycylalanylphenylalaninmethylester*

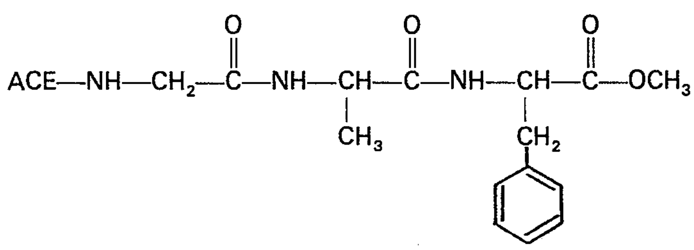

analog Beispiel 19 mit Glycylalanylphenylalaninmethylesterhydrochlorid; Fp. 145 bis 147°C (aus Isopropanol); Ausbeute 83% der Theorie.

$[\alpha]_D^{20} = -7{,}2$ (c = 0,5; Dimethylformamid).

Für $C_{36}H_{37}ClN_4O_9$

| | | | | |
|---|---|---|---|---|
| berechnet: | C 61,32%, | H 5,29%, | Cl 5,03%, | N 7,95%; |
| gefunden: | C 60,82%, | H 5,38%, | Cl 5,19%, | N 8,32%. |

Beispiel 21

a) *N-α-Formyl-N-γ-benzyloxycarbonyldiaminobutyrylphenylalaninhydrazid*

53 g (0,12 Mol) N-α-Formyl-N-γ-benzyloxycarbonyldiaminobutyrylphenylalaninmethylester werden bei 40°C in 400 ml Methanol gelöst und mit 10 g (ca. 0,16 Mol) 80 %igem Hydrazinhydrat versetzt. Nach 24-stündigem Stehen bei Raumtemperatur wird der ausgefallene Niederschlag abgesaugt, die Mutterlauge auf 50 ml eingeengt und erneut zur Kristallisation gebracht.

Fp. 205 bis 207°C (aus wäßrigem Dimethylformamid);

Ausbeute 50 g = 94% der Theorie;

$[\alpha]_D^{20} = 11{,}5$ (c = 2; Dimethylformamid).

Für $C_{22}H_{27}N_5O_5$

berechnet: C 62,57%, H 6,17%, N 9,52%;
gefunden: C 62,46%, H 6,15%, N 9,65%.

b) *N-α-Formyl-N-γ-benzyloxycarbonyldiaminobutyrylphenylalanylphenylalaninmethylester*

88,3 g (0,2 Mol) der vorstehend unter a) beschriebenen Verbindungen werden unter Erwärmen in 1000 ml Dimethylformamid gelöst und mit 20 ml Eisessig und 400 ml nHCl vermischt. Nach Abkühlen auf −10°C werden zunächst langsam 14,4 g (0,208 Mol) Natriumnitrit in 60 ml Wasser und anschließend eine vorgekühlte Lösung von 35,8 g (0,02 Mol) L-Phenylalaninmethylesterhydrochlorid, gelöst in 200 ml Dimethylformamid, hinzugefügt. Die Reaktionslösung wird mit etwa 54 g Triethylamin neutralgestellt und 24 Stunden im Kühlschrank aufbewahrt. Nach Aufschlämmen mit doppelter Menge Wasser wird der ausgefallene Niederschlag abfiltriert, mit n Salzsäure und Wasser gewaschen, getrocknet und aus Methanol rekristallisiert.

Fp. 179 bis 181°C, Ausbeute 106 g = 92% der Theorie;
$[\alpha]_D^{20} = -22,5$ (c = 2; Dimethylformamid).

Für $C_{32}H_{36}N_4O_7$

berechnet: C 65,29%, H 6,16%, N 9,52%;
gefunden: C 65,26%, H 6,33%, N 9,61%.

c) N-γ-Benzyloxycarbonyldiaminobutyrylphenylalanylphenylalaninmethylesterhydrochlorid

52 g (0.0885 Mol) der vorstehend unter b) beschriebenen Verbindung werden in 150 ml Methanol und 35 ml n methanolischer HCl suspendiert und 20 Stunden bei Raumtemperatur gerührt. Die nunmehr klare Lösung wird eingedampft und der Rückstand aus Methanol/Ether rekristallisiert.

Fp. 220 bis 222°C, Ausbeute 29 g (= 54% der Theorie).
$[\alpha]_D^{20} = +1,65$ (c = 2; Dimethylformamid).
Für $C_{31}H_{37}ClN_4O_6 . 1/2\ H_2O$

berechnet: C 61,43%, H 6,32%, Cl 5,85%, N 9,32%;
gefunden: C 61,26%, H 6,60%, Cl 5,53%, N 9,27%.

d) N-α-ACE—N-γ-Benzyloxycarbonyldiaminobutyrylphenylalanylphenylalaninmethylester

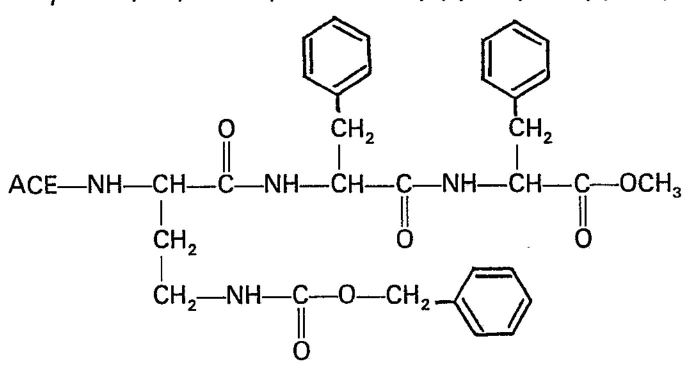

analog Beispiel 19 aus vorstehend unter c) beschriebener Verhindung und ACE-2,4,5-Trichlorphenylester.

Fp. 224 bis 227°C (aus Dioxan/Ether 2:1); Ausbeute 69% der Theorie.
$[\alpha]_D^{20} = -20,6$ (c = 0,5; Dimethylformamid).
Für $C_{52}H_{52}ClN_5O_{11}$

berechnet: C 65,16%, H 5,47%, Cl 3,70%, N 7,31%;
gefunden: C 64,72%, H 5,63%, Cl 3,96%, N 7,17%.

**Patentansprüche**

1. Indolylacetoxyacetylaminoessigsäurederivate der allgemeinen Formel (I)

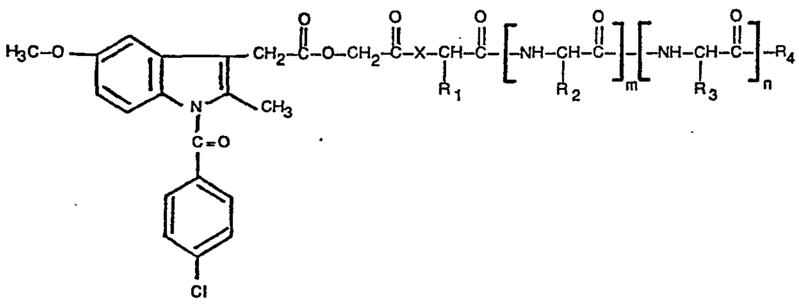

(I)

in welcher

m und n für die Ziffern 0 oder 1 stehen,

X für eine —NH— oder —$OCH_2$-Gruppe steht,

$R_1$, $R_2$ und $R_3$ gleich oder verschieden sind und jeweils für Wasserstoff, eine gegebenenfalls durch Hydroxy, Alkoxy mit 1 bis 4 Kohlenstoffatomen, Alkylthio mit 1 bis 4 Kohlenstoffatomen oder durch Phenalkyloxycarbonylamino mit 1 bis 4 Kohlenstoffatomen im Alkylteil, substituiertes Alkyl mit 1 bis 5 Kohlenstoffatomen oder für eine Phenyl- oder Phenalkylgruppe mit 1 bis 4 Kohlenstoffatomen im Alkylteil, wobei die Phenylringe gegebenenfalls durch Trifluormethyl, Alkoxy mit 1 bis 4 Kohlenstoffatomen, Hydroxy oder Halogen substituiert sind oder für eine Heterocyclusmethylgruppe mit 5 bis 6 Ringgliedern, in welcher 1 oder 2 Glieder durch Stickstoff ersetzt sein können und an welche gegebenenfalls ein Benzolring annelliert ist, stehen oder für den Fall, daß m und n jeweils 0 bedeuten $R_1$ zusätzlich für eine Aminogruppe, eine Alkoxycarbonylaminogruppe oder eine Phenalkoxycarbonylaminogruppe mit jeweils 1 bis 5 Kohlenstoffatomen im Alkoxyteil steht,

$R_4$ für Hydroxy, Alkoxy mit 1 bis 4 Kohlenstoffatomen, Phenalkoxy mit 1 bis 4 Kohlenstoffatomen im Alkoxyteil, Amino, Alkylamino mit 1 bis 4 Kohlenstoffatomen im Alkylteil, Benzylamino oder Phenylamino steht,

sowie ihre gegenbenenfalls mit Säuren und Basen gebildeten physiologisch verträglichen Salze.

2. Verfahren zur Herstellung von Indolacetoxyacetylaminoessigsäurederivate der allgemeinen Formel (I) gemäß Anspruch 1, dadurch gekennzeichnet, daß man

a) [1-(4-Chlorbenzoyl)-5-methoxy-2-methyl-3-indolyl]-acetoxyessigsäure (nachfolgend als ACE—OH bezeichnet) mit Chlorameisensäureestern der allgemeinen Formel

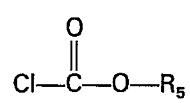

(II)

in welcher

$R_5$ für eine Alkylgruppe mit 1 bis 5 Kohlenstoffatomen steht,

zu gemischten Anhydriden der allgemeinen Formel (III)

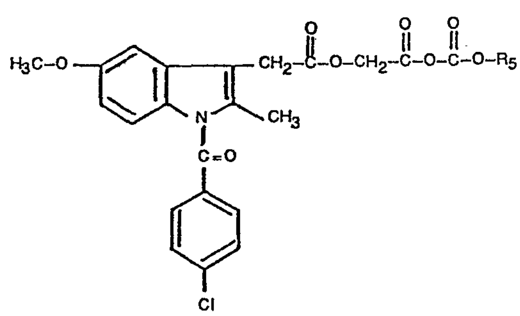

(III)

in welcher

$R_5$ die oben angegebene Bedeutung hat,

umsetzt und diese mit Verbindungen der allgemeinen Formel (IV)

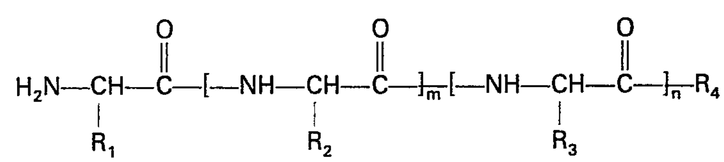

(IV)

in welcher

m, n und $R_1$ bis $R_4$ die in Anspruch 1 angeführte Bedeutung haben, zur Reaktion bringt, oder

b) die Verbindung ACE—OH mit einem entsprechend substituierten Phenol zu einem aktivierten Ester der allgemeinen Formel (V)

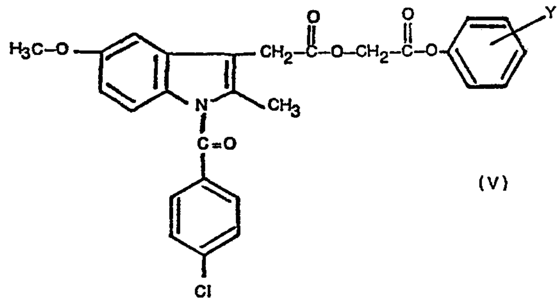

(V)

in welcher

Y für 2 bis 5 Halogenatome oder 1 bis 2 Nitrogruppen steht,

kondensiert und mit Verbindungen der allgemeinen Formel (IV) umsetzt oder

c) für den Fall, daß Hydroxylgruppen enthaltende Aminosäurederivate esterartig verknüpft werden sollen, die Verbindung ACE—OH mit Verbindungen der allgemeinen Formel (VI)

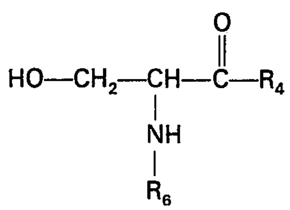

(VI)

in welcher

$R_4$ die oben angeführte Bedeutung hat und

$R_6$ für eine in der Peptidchemie übliche Aminoschutzgruppe steht,

umsetzt und anschließend den Rest $R_6$ bekannter Weise abspaltet und gegebenenfalls die so erhaltenen Verbindungen der allgemeinen Formel (I) mit säuren und Basen in die physiologisch verträglichen Salze verwandelt.

3. Arzneimittel enthaltend mindestens ein Indolylacetoxyacetylaminoessigsäure-Derivat gemäß Anspruch 1.

**Claims**

1. Indolylacetoxyacetylaminoacetic acid derivatives of the general formula (I)

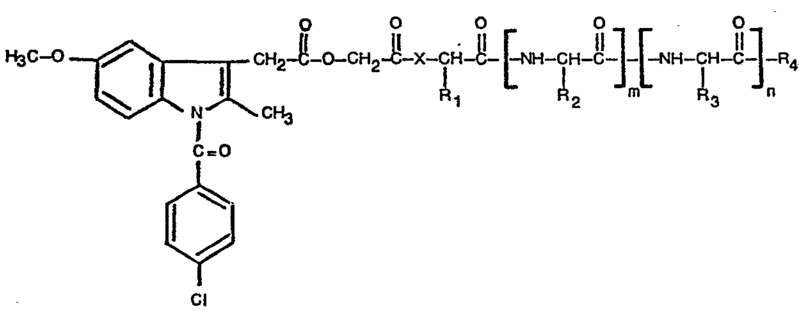

(I)

in which

m and n represent the numbers 0 or 1,

X represents a —NH— or —$OCH_2$— group,

$R_1$, $R_2$ and $R_3$ are identical or different and each represent hydrogen, an alkyl with 1 to 5 carbon atoms optionally substituted by hydroxy, alkoxy with 1 to 4 carbon atoms, alkylthio with 1 to 4 carbon atoms or by phenalkyloxycarbonylamino with 1 to 4 carbon atoms in the alkyl part, or represents a phenyl or phenalkyl group with 1 to 4 carbon atoms in the alkyl part, the phenyl rings optionally being substituted by trifluoromethyl, alkoxy with 1 to 4 carbon atoms, hydroxy or halogen or represents a heterocyclic methyl group with 5 to 6 ring members, in which 1 or 2 members can be replaced by nitrogen and to which a benzene ring is optionally fused, or in the case where m and n each represent O, $R_1$ additionally represents an amino group, an alkoxycarbonylamino group or a phenalkoxycarbonylamino group with in each case 1 to 5 carbon atoms in the alkoxy part,

$R_4$ represents hydroxy, alkoxy with 1 to 4 carbon atoms, phenalkoxy with 1 to 4 carbon atoms in the alkoxy part, amino, alkylamino with 1 to 4 carbon atoms in the alkyl part, benzylamino or phenylamino,

and their physiologically acceptable salts appropriately formed with acids and bases.

2. Process for the preparation of indolylacetoxyacetylaminoacetic acid derivatives of the general formula (I) according to Claim 1, characterised in that

a) [1-(4-chlorobenzoyl)-5-methoxy-2-methyl-3-indolyl]acetoxyacetic acid (designated ACE—OH in the following) is reacted with chloroformic acid esters of the general formula

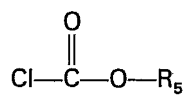
(II)

in which

$R_5$ represents an alkyl group with 1 to 5 carbon atoms,

to give mixed anhydrides of the general formula (III)

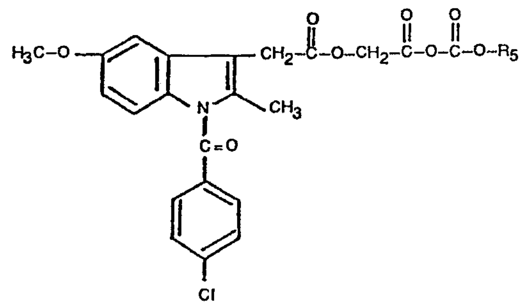
(III)

in which

$R_5$ has the meaning indicated above,

and these are reacted with compounds of the general formula (IV)

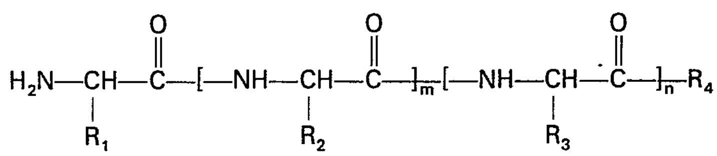
(IV)

in which

m, n and $R_1$ to $R_4$ have the meaning indicated in Claim 1, or

b) the compound ACE—OH is subjected to a condensation reaction with an appropriately substituted phenol to give an activated ester of the general formula (V)

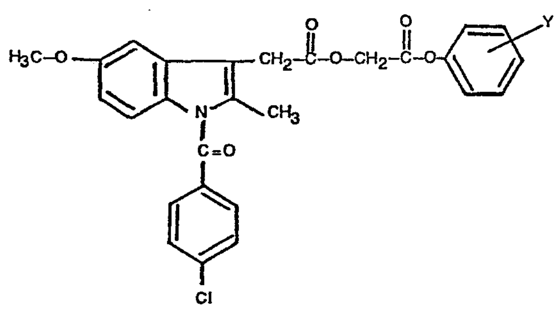
(V)

in which

Y represents 2 to 5 halogen atoms or 1 to 2 nitro groups,

and the activated ester is reacted with compounds of the general formula (IV) or

c) in the case where aminoacid derivatives containing hydroxyl groups are to be linked in an ester-like manner, the compound ACE—OH is reacted with compounds of the general formula (VI)

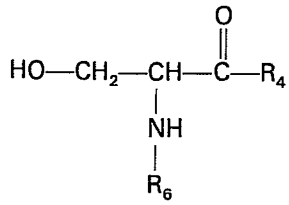

(VI)

in which

$R_4$ has the meaning indicated above and

$R_6$ represents an amino-protective group which is customary in peptide chemistry,

and the radical $R_4$ is then split off in a known manner, and the compounds of the general formula (I) thus obtained are optionally converted into the physiologically acceptable salts using acids and bases.

3. Medicaments containing at least one indolylacetoxyacetylaminoacetic acid derivative according to Claim 1.

**Revendications**

1. Dérivés d'indolylacétoxyacétylaminoacides de formule générale (I)

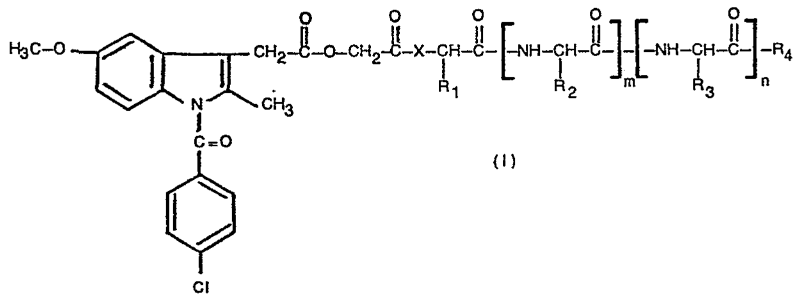

(I)

dans laquelle

m et n représentent les chiffres 0 or 1,

X représente un groupe —NH— ou —$OCH_2$—,

$R_1$, $R_2$ et $R_3$ sont identiques ou différents et représentent chacun l'hydrogène, un alkyle en $C_1$—$C_5$ éventuellement substitué par hydroxy, alcoxy en $C_1$—$C_4$, alkylthio en $C_1$—$C_4$ ou par un phénylalcoxycarbonylamino contenant 1 à 4 atomes de carbone dans la partie alkyle, ou un groupe phényle ou phénylalkyle contenant 1 à 4 atomes de carbone dans la partie alkyle, les noyaux phényle étant éventuellement substitués par trifluorométhyle, alcoxy en $C_1$—$C_4$, hydroxy ou halogène, ou un groupe hétérocycle-méthyle à 5 ou 6 chaînons, dans lequel 1 ou 2 chaînons peuvent être remplacés par l'azote et sur lequel est éventuellement condensé un noyau benzénique, ou bien, dans le cas où m et n sont égaux chacun à 0, $R_1$ représente en outre un groupe amino, un groupe alcoxycarbonylamino ou un groupe phénylalcoxycarbonylamino contenant chaque fois 1 à 5 atomes de carbone dans la partie alcoxy,

$R_4$ représente un groupe hydroxy, alcoxy en $C_1$—$C_4$, phénylalcoxy contenant 1 à 4 atomes de carbone dans la partie alkyle, benzylamino ou phénylamino,

ainsi que leurs sels physiologiquement compatibles formés avec des acides et des bases.

2. Procédé pour la préparation de dérivés d'indoleacétoxyacétylaminoacides de formule générale (I) selon la revendication 1, caractérisé en ce que:

a) on fait réagir l'acide [1-(4-chlorobenzyl)-5-méthoxy-2-méthyl-3-indolyl]-acétoxyacétique (ci-après dénommé ACE—OH) avec des esters chloroformiques de formule générale

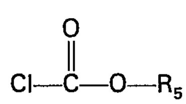

(II)

$R_5$ représente un groupe alkyle en $C_1$—$C_5$,

en anhydrides mixtes de formule générale (III)

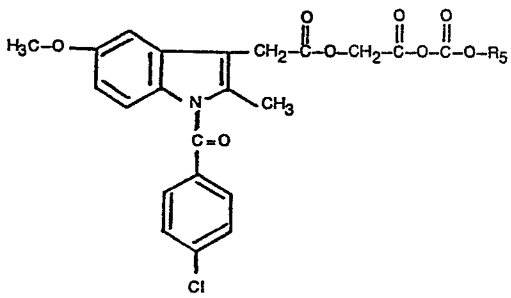

(III)

dans laquelle

$R_5$ a la signification indiquée ci-dessus,

et on fait réagir ceux-ci avec des composés de formule générale (IV)

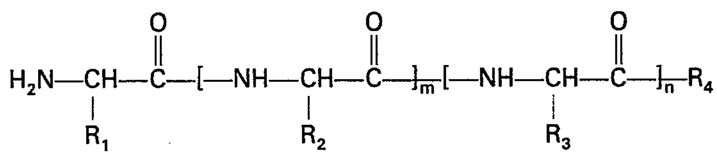

(IV)

dans laquelle

m, n et $R_1$ à $R_4$ ont la signification indiquée dans la revendication 1, ou bien

b) on condense le composé ACE—OH avec un phénol substitué correspondant en un ester activé de formule générale (V)

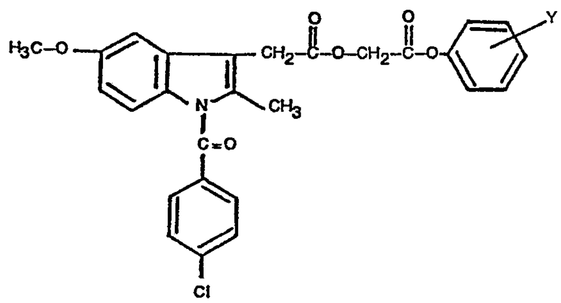

(V)

dans laquelle

Y représente 2 à 5 atomes d'halogène ou 1 ou 2 groupes nitro et on fait réagir avec des composés de formule générale (IV), ou bien

c) dans le cas où les dérivés d'aminoacides contenant des groupes hydroxy doivent être liés sous forme ester, on fait réagir le composé ACE—OH avec des composés de formule générale (VI)

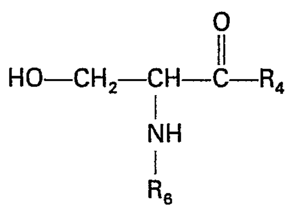

(VI)

dans laquelle

$R_4$ a la signification indiquée ci-dessus et

$R_6$ représente un groupement protecteur du reste amino habituel dans la chimie des peptides, et on élimine ensuite le reste $R_6$ de manière connue et, éventuellement, on transforme les composés de formule générale (I) ainsi obtenus avec des acides et des bases en les sels physiologiquement compatibles.

3. Médicaments contenant au moins un dérivé d'indolylacétoxyacétylaminoacide selon la revendication 1.